# EUROPEAN PATENT APPLICATION

(11) **EP 2 371 805 A1**
(43) Date of publication of application: **05.10.2011**
(21) Application number: 11167801.7
(22) Date of filing: 20.10.2006
(51) Int. Cl.: C07C 67/02, C07C 67/08, C08G 63/78, C07C 69/003, C07C 69/007, C08G 63/60, C11B 13/00

(54) **Method for the manufacture of oligo- and polyesters from a mixture of carboxylic acids obtained from suberin and/or cutin and the use thereof**

(30) Priority: 21.10.2005 FI 20055568
(62) Divisional of application: 06820088.0
(71) Applicant: VALTION TEKNILLINEN TUTKIMUSKESKUS, 02044 VTT (FI)
(72) Inventor: Koskimies, Salme, 00850 Helsinki (FI); Hulkko, Janne, 08200 Lohja (FI); Pitkänen, Pauliina, 02730 Espoo (FI); Heiskanen, Nina, 00510 Helsinki (FI); Yli-Kauhaluoma, Jari, 00790 Helsinki (FI); Wähälä, Kristiina, 00130 Helsinki (FI)
(74) Representative: Papula Oy

(57) **Abstract**

The invention relates to a method for processing mixtures of carboxylic acids obtained as hydrolysis products of suberin and cutin, particularly suberin and cutin isolated from birch bark, to give mono-, oligo- and polyesters, or corresponding ester-ethers, as well as the use of the products thus obtained as lubricants, fuel components, plasticizers, surface active agents, environmentally friendly agents for modifying wood, binders in coatings, adhesives, printing inks and composites, further in various cosmetic applications.

## Description

### Field of the invention

The invention relates to mono-, oligo- and polyesters of mixtures of carboxylic acids obtained as hydrolysis products of suberin and cutin found in plants, especially in the cuticle of various tree species, and further to corresponding ester-ethers, particularly to a method for producing oligo- and polyesters from mixtures of carboxylic acids derived from suberin and/or cutin, and moreover to the use of said oligo- and polyesters obtained by the method as lubricants, fuel components, plasticizers, surface active agents, environmentally friendly agents for modifying wood, binders in coatings, in adhesives, printing inks and composites, further in various cosmetic applications and as starting materials for resins.

### Prior art

Suberin is a natural biopolymer typically found in cell walls of plants. Considerable amounts of suberin, typically about 30 - 60 % by weight, are present in various plant species such as potato and cotton, and particularly in the cuticle portion of cork oak, beech, douglas fir, and in birch bark. Another natural biopolymer, cutin, is structurally very closely related to suberin, and is typically found outside cell walls of almost all plants. Cutin differs from suberin only in that it contains very low amounts of aromatic compounds.

Suberin and cutin are typically separated from plant material by extracting so-called extractable constituents with an organic solvent such as acetone, alcohols and the like, followed by separation of the poorly soluble polymeric suberin and/or cutin by filtration. Suberin and/or cutin thus obtained may further be depolymerized by hydrolysis using known methods such as acidic or basic hydrolysis or with alkaline fusion to give aliphatic monomers, mainly carboxylic acids and phenolic derivatives, as reported by Ekman, R. et al., Paperi ja Puu (1985) 67 (4), 255 - 273), Graca J., et al., Holzforschung (1999) 54 (4), 397 - 402, and Kolattukudu, P.E., Science 208 (1980) 990 - 1000.

Carboxylic acid distributions, especially fatty acid distributions, of the hydrolysis products of suberin and cutin somewhat differ from each other and also depend on the hydrolysis method and plant starting materials used. Fatty acid mixtures obtained from cutin and suberin with known hydrolysis methods are presented below. Tables 1 and 2 below show the monomeric distributions obtained from suberin of birch bark respectively by alkaline hydrolysis using alkali metal hydroxide (NaOH/iPrOH), and by alkaline treatment (alkali fusion KOH/330 °C).

**Table 1**

| Carboxylic acids of birch suberin (NaOH/iPrOH) | |
|---|---|
| **Monomer** | **% by weight** |
| HOOC(CH2)₇CH=CH(CH₂)₇COOH | 5 |
| HOCH₂(CH₂)₇CH=CH(CH₂)₇COOH | 12 |
| 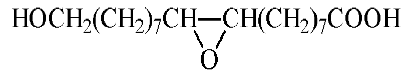 | 39 |
| 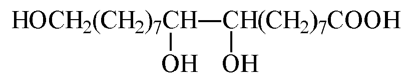 | 8 |
| HOCH₂(CH₂)₂₀COOH | 14 |
| HOOC(CH₂)₂₀COOH | 8 |
| HOCH₂(CH₂) ₆CHOH(CH₂)₇COOH | 3 |

**Table 2**

| Carboxylic acids of birch suberin (KOH/ 330 °C) | |
|---|---|
| **Monomer** | **% by weight** |
| CH₃(CH₂)₆COOH | 14 |
| HOOC(CH₂)₇COOH | 25 |
| HOOC(CH₂)₁₄COOH | 15 |
| HOOC(CH₂)₂₀COOH | 21 |

The distribution of monomers in the alkaline hydrolysis product of cutin is shown in Table 3.

**Table 3**

| Carboxylic acids of cutin |
|---|
| **C16-family (y = 8, 7, 6 or 5 and x + y = 13):** |
| CH₃(CH₂)₁₄COOH |
| HOCH₂(CH₂)₁₄COOH |
| HOCH₂(CH₂)ₓCHOH(CH₂) _{y}COOH |

| **C 18-family :** |
|---|
| CH₃(CH₂)₇CH=CH(CH₂)₇COOH |
| HOCH₂(CH₂)₇CH=CH(CH₂)₇COOH |
| 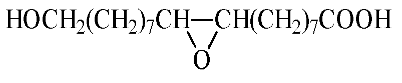 |
| 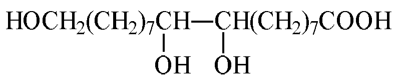 |

Chemical processing of the carboxylic acids and particularly mixtures of carboxylic acids obtained from suberin and cutin to give industrial products is at present rather poorly known. Processing of suberin obtained from the bark of cork oak to give polyurethane polymers is disclosed by Pascoal Neto et al., Industrial Crops and Products (1997) 6 (2), 163 - 167, and (1999) 10(1), 1 -10.

Birch is used as raw material by pulp industry where external bark is peeled off the trunk and at present mainly utilized as energy by burning. It would be possible to extract carboxylic acids as hydrolysis products of suberin and cutin in amounts of about 4000 tons/a by each birch pulp production unit. These carboxylic acids are so far not utilized in the industry.

Products typically used in lubricant, fuel component, plasticizer, binder, coating, composite applications, and as environmentally friendly agents for modifying wood are produced from carboxylic acids as pure compounds obtained by processing hydrocarbons of vegetable oil or fossil origin. Carboxylic acid compositions of these products clearly differ from that obtained by hydrolysis of suberin or cutin.

### Objects of the invention

The object of the present invention is to make use of the renewable natural suberin and cutin raw materials particularly abundant in the bark of some tree species, and to develop a simple and industrially feasible method for producing oligo- and polyesters of mixtures of carboxylic acids obtained from suberin and cutin.

The invention is directed to a method for esterification of mixtures of aliphatic C14 - C24 carboxylic acids obtained from suberin and/or cutin by hydrolysis.

The invention is directed to a method for esterification of mixtures of aliphatic C14 - C24 carboxylic acids obtained from suberin and/or cutin by hydrolysis, and for transesterification of the product thus obtained.

The invention is also directed to a method for converting esters of mixtures of aliphatic C14 - C24 carboxylic acids obtained from suberin and/or cutin by hydrolysis to give mono-, oligo- and polyesters.

The invention is also directed to the use of the mono-, oligo- and polyesters of mixtures of aliphatic C14 - C24 carboxylic acids obtained from suberin and/or cutin by hydrolysis in lubricant, fuel component, plasticizer, binder, coating, composite, adhesive, printing ink, and cosmetic applications and further as agents for modifying wood, and as surface active agents.

The characteristic features of the methods and use of the invention are presented in the Claims.

### Summary of the invention

The invention relates to a method for processing mixtures of aliphatic C14 - C24 carboxylic acids, typically mixtures of fatty acids obtained as hydrolysis products of suberin and cutin, particularly suberin and cutin extracted by known methods from birch bark, to give mono-, oligo- and polyesters, or corresponding ester-ethers, as well as to the use of the products thus produced as lubricants, fuel components, plasticizers, surface active agents, environmentally friendly agents for modifying wood, binders in coatings, in adhesives, printing inks and composites, and further in cosmetic applications.

Aliphatic C14 - C24 carboxylic acids refer here to C14 - C24 carboxylic acids, hydroxy acids, dicarboxylic acids, and C14 - C24 carboxylic acids also having a double bond and/or a epoxy group.

Mixtures of aliphatic C14 - C24 carboxylic acids refer here to mixtures comprising from 0 to 30 %, preferably from 5 to 20 %, by weight, of monoacids, at least 5 %, preferably from 10 to 50 %, by weight, of diacids, preferably from 10 to 30 %, by weight of hydroxy acids, and from 0 to 50 %, preferably from 10 to 40 %, by weight of diacids or hydroxy acids having a double bond and/or a epoxy group.

### Detailed description of the invention

It was surprisingly found that environmentally friendly esters and ester-ethers may be produced from mixtures of aliphatic carboxylic acids obtained as hydrolysis products of suberin and/or cutin, and further, they may be used as starting materials in the production of oligo/polyester products with industrially significant feasibility potential for various applications.

The biopolymer selected from the group consisting of suberin, cutin, and the mixtures thereof is hydrolysed using prior art methods, such as acid or alkaline hydrolysis, in the presence of an alkali metal hydroxide and a solvent (e.g. NaOH/iPrOH), or using alkaline fusion in the presence of an alkali metal hydroxide, at elevated temperatures (e.g. alkaline fusion KOH/330 °C). Mixtures of aliphatic C14 - C24 carboxylic acids are typically obtained as hydrolysis products. Preferably, these mixtures of aliphatic C14 - C24 carboxylic acids are directly used as starting compounds in the method of the invention without purification/separation steps.

In the method of the invention, the mixtures of aliphatic C14 - C24 carboxylic acids obtained by hydrolysis of suberin and/or cutin are directly esterified without any purification/separation steps. Esterification may be carried out in three alternative ways.

In accordance with the first embodiment a) of the invention, the mixtures of C14-C24 carboxylic acids obtained by hydrolysis of suberin and/or cutin are first subjected to esterification/etherification, followed by the transesterification of the product thus obtained with an alcohol. Said esterification/etherification is preferably carried out as a so-called *in situ* esterification/etherification. According to the second embodiment b), mixtures of C14 - C24 carboxylic acids obtained by hydrolysis of suberin and/or cutin are directly subjected to said esterification reaction with a monocarboxylic acid or a mixture of monocarboxylic acids, or with a mixture of plant oil fatty acids. According to the third embodiment c), a mixture of C14 - C24 carboxylic acids obtained by hydrolysis of suberin and/or cutin is first reacted with an carboxylic anhydride, followed by esterification of the product thus obtained.

More precisely, mixtures of aliphatic C14 - C24 carboxylic acids obtained from suberin and/or cutin by hydrolysis are esterified according to any of the embodiments a), b), or c) of the method of the invention, the embodiment
a) comprising the reaction of the mixtures of aliphatic C14 - C24 carboxylic acids obtained from suberin and/or cutin by hydrolysis, or corresponding mixtures of alkali metal salts of the carboxylic acids with dimethyl sulphate in the presence of a base to give a mixture of ethers/esters of C14 - C24 carboxylic acids, followed by the transesterification of said ether/ester mixture by reaction with at least one linear or branched alcohol selected from the group consisting of C3-C18 monoalcohols, oligoalcohols, polyols, mixtures of monoalcohols and polyols, and mixtures of monoalcohols and oligoalcohols, in the presence of a base catalyst; or
b) comprising the reaction of the mixtures of aliphatic C14 - C24 carboxylic acids obtained from suberin and/or cutin by hydrolysis with at least one saturated or unsaturated linear or branched C2-C18 monocarboxylic acid, or with a mixture of monocarboxylic acids, or with a plant oil fatty acid or with a mixture of said fatty acids in the presence of an acid catalyst; or
c) comprising the reaction of the mixtures of aliphatic C14 - C24 carboxylic acids obtained from suberin and/or cutin by hydrolysis with at least one saturated or unsaturated, cyclic, aromatic or linear carboxylic anhydride to give a mixture of oligoacids, followed by the reaction thereof with at least one linear or branched alcohol selected from the group consisting of C3-C18 monoalcohols, oligoalcohols, and mixtures of monoalcohols and oligoalcohols, in the presence of an acid catalyst.

The *in situ* esterification/etherification according to the embodiment a) of the invention may be represented by Reaction 1 shown in the simplified scheme below, where a mixture of alkali metal salts of aliphatic carboxylic acids, or a corresponding acid mixture obtained by e.g. acid or basic hydrolysis of suberin and/or cutin is reacted with dimethyl sulphate to yield the desired esterification/etherification product. Only the main component of the mixture is shown in the scheme.

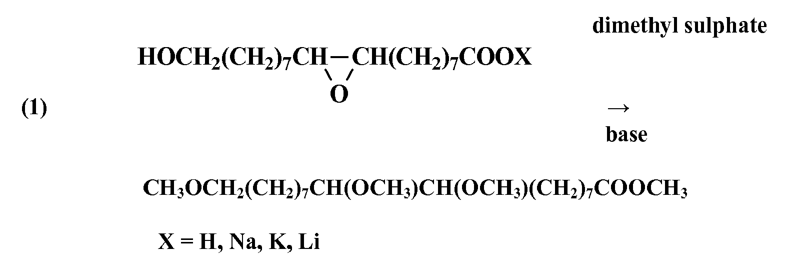

In the embodiment according to Reaction 1, (0.5 - 2 mols, preferably 1-1.5 mols) of a mixture of alkali metal salts of aliphatic C14 - C24 carboxylic acids, the alkali metal being Na, K, or Li, or a corresponding mixture of free carboxylic acids obtained by hydrolysis of suberin and/or cutin, are reacted with dimethyl sulphate (0.5 - 4 mols, preferably 1 - 3 mols) in an inert solvent, preferably in acetone, methylethylketone, cyclohexanone or tetrahydrofuran, or in mixtures thereof, at a temperature of 20 - 100 °C, preferably at 30 - 60 °C, for 1 - 12 hours, preferably for 2 - 6 hours, in the presence of a base, the base being selected from the group consisting of NaOH, KOH, LiOH, NaOMe, KOMe, K₂CO₃, the preferable base being NaOH or K₂CO₃. Unreacted dimethyl sulphate is decomposed with water. The solvent is removed by a suitable method, such as evaporation, and the esterification/etherification product of the carboxylic acid mixture is purified and dried.

The ester/ether mixture obtained with Reaction 1 shown above serves as the starting material in the production of various mono-, oligo- or polyester products by transesterification.

Transesterification may be carried out according to Reactions 2, 3 or 4 shown below. Representative main components are shown in the reaction scheme for demonstrating the ester selectivity.

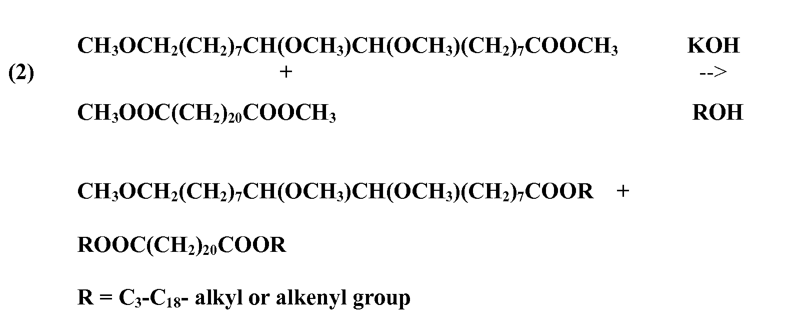

According to Reaction 2, the mixture of carboxylic acid ethers/esters obtained in Reaction 1 are reacted with at least one linear or branched alcohol selected from the group consisting of C3-C18 monoalcohols ROH, oligoalcohols (HO)ₘR, where m = 2 - 6, polyols, mixtures of mono- and oligoalcohols, and mixtures of monoalcohols and polyols, in the presence of a basic catalyst, preferably KOH or NaOH, at a temperature of 20 - 120 °C, preferably at 40 - 100 °C. The monoalcohol is preferably 1-, 2- or i-butanol, 1- or i-valeric alcohol, 1-hexanol or 2-ethyl hexanol. The oligoalcohol (HO)ₘR is preferably a C2-C6-diol. The polyol is selected from the group consisting of ethyleneglycol, di- and oligoethyleneglycol, propyleneglycol, di- and oligopropyleneglycol, 1,3-methylpropanediol, neopentylglycol, trimethylolpropane, pentaerythritol, dipentaerythritol, 1,4-butanediol and 1,6-hexanediol. After neutralization, catalytic residues and the excessive alcohol are removed by washing with water, and for instance by vacuum distillation respectively.

Transesterification may also be carried out according to Reaction 3 shown below.

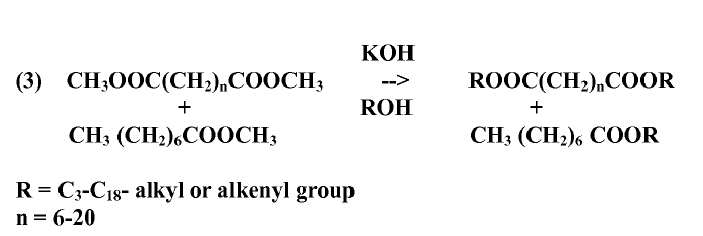

In Reaction 3, the mixture of methyl esters of carboxylic acids obtained from suberin or respectively from cutin by alkaline fusion (KOH/330 °C), followed by esterification (preferably using an *in situ* method) is reacted with at least one linear or branched alcohol selected from the group consisting of C3-C18 monoalcohols ROH, oligoalcohols (HO)ₘR, where m = 2 - 6, polyols, mixtures of mono- and oligoalcohols, and mixtures of monoalcohols and polyols, in the presence of a basic catalyst, preferably KOH or NaOH, at 20 - 120 °C, preferably at 40 - 100 °C. The monoalcohol is preferably 1-, 2- or i-butanol, 1- or i-valeric alcohol, 1-hexanol or 2-ethyl hexanol. The oligoalcohol is preferably a C2-C6-diol. The polyol is selected from the group consisting of ethyleneglycol, di- and oligoethyleneglycol, propyleneglycol, di- and oligopropyleneglycol, 1,3-methylpropanediol, neopentylglycol, trimethylolpropane, pentaerythritol, dipentaerythritol, 1,4-butanediol and 1,6-hexanediol. After neutralization, catalytic residues and the excessive alcohol/polyol are removed by washing with water, and for instance by vacuum distillation, respectively.

A liquid viscous product having a boiling point range from 250 to 450 °C is typically obtained from Reactions 2 and 3 at room temperature. The product may be used as a lubricant for instance in engines, in oils for refrigerating devices and in other machines and apparatuses, and further as a so-called lubricating additive in fuels, as a plasticizer, surface active agent, as a medium in cosmetic applications, as a compatibility improver for producing mixtures of plastics such as blends or composites, as an agent for modifying wood, such as an impregnating agent, as a binder in coatings and printing inks, either as such or in combination with products produced using known techniques.

At least one monoalcohol ROH or an oligoalcohol (OH)ₖR such as ethyleneglycol (EG), propyleneglycol (PG), dimers and oligomers of ethylene- and propyleneglycols, neopentylglycol (NPG), trimethylolpropane (TMP), pentaerythritol (PE), dipentaerythritol (di-PE), and the like may also be added to the *in situ* reaction product according to the first embodiment of the method of the invention, and the transesterification may be carried out according to Reaction 4 shown below with at least one monoalcohol and at least one oligoalcohol.

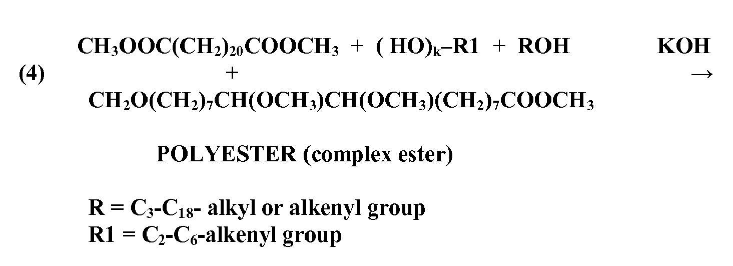

In this case, according to Reaction 4, the mixture of carboxylic acid ethers/esters obtained from the *in situ* esterification/etherification reaction is reacted with at least one linear or branched C3-C18 monoalcohol ROH, preferably with 1-, 2- or i-butanol, 1- or i-valeric alcohol, 1-hexanol or 2-ethylhexanol, and at least with one oligoalcohol (HO)ₖR, where k ≥ 2, said oligoalcohol being selected from the group consisting of ethyleneglycol, di- and oligoethyleneglycol, propyleneglycol, di- and oligopropyleneglycol, neopentylglycol, trimethylolpropane, pentaerythritol, dipentaerythritol, 1,4-butanediol and 1,6-hexanediol, in the presence of a basic catalyst, preferably KOH or NaOH, at 20 - 120 °C, preferably at 40 - 100 °C. After neutralization, catalytic residues and the excessive alcohol/oligoalcohol are removed by washing with water, and for instance by vacuum distillation, respectively.

A typically viscous liquid or waxy product at room temperature, having a low melting temperature, Tg < 70 °C, is obtained, said product being useful as a lubricant for instance in engines, in oils for refrigerating devices, and in other machines and apparatuses, and further as a so-called lubricating additive in fuels, as a plasticizer, surface active agent, as a compatibility improver for producing mixtures of plastics such as blends or composites, as an agent for modifying wood, such as an impregnating agent, as a binder in coatings and printing inks, either as such or in combination with products produced using known techniques.

According to the embodiment b) of the invention, the esterification is directly performed with the fatty acid mixture of suberin and/or cutin according to Reaction 5 shown below.

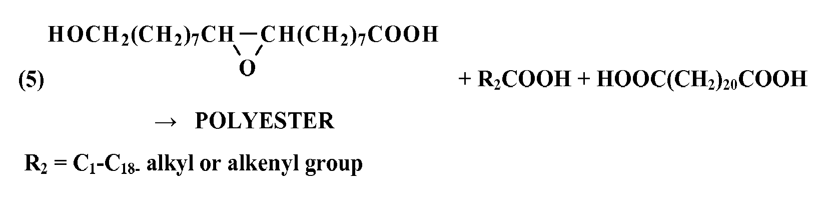

In the embodiment according to Reaction 5, the mixture of aliphatic C14 - C24 carboxylic acids obtained from suberin and/or cutin by hydrolysis is reacted with at least one saturated or unsaturated linear or branched C2 - C18 monocarboxylic acid or with a mixture of corresponding monocarboxylic acids, preferably with 1- or i-butyric acid, 1- or i-valeric acid, caprylic acid, 2-ethylhexyl acid, dodecyl acid, myristinic acid, stearic acid, or with oleic acid, or with a mixture of fatty acids, preferably a mixture of fatty acids from linenseed oil, soybeen oil, tall oil or rapeseed oil, either as such or in a solvent selected from the group consisting of toluene, xylene and mixtures thereof, in the presence of a protonic acid catalyst such as H₂SO₄, MeSO₃H, HCl, H₃PO₄, p-toluene sulphonic acid or Lewis acid e.g. titanates, (RO)₄Ti or stannous oxide (SnO), at a temperature of 60 - 280 °C, preferably at 100 - 260 °C, in an inert atmosphere, preferably in a nitrogen or argon atmosphere, for 1 - 24 hours, preferably for 2 - 12 h. The mixture is cooled, washed, the solvent and residual monomers are removed for instance by vacuum distillation, followed by drying of the ester product.

A typically viscous liquid or waxy product at room temperature, having a low melting temperature, Tg < 100 °C, is obtained, said product being useful as a lubricant for instance in engines, in oils for refrigerating devices and in other machines and apparatuses, and further, as a plasticizer, as a medium in cosmetics, as a compatibility improver for producing mixtures of plastics such as blends or composites, and as an agent for modifying wood, such as an impregnating agent, as a binder in coatings and printing inks, either as such or in combination with products produced using known techniques. Typical application fields of the polyester produced according to Reaction 5 are as binders in paints, printing inks and composites either as such or in combination with conventional products by using the fatty acid mixture of suberin and/or cutin as the starting material with other carboxylic acids and polyols, or by blending the product obtained from Reaction 5 with conventional binders.

In accordance with the embodiment c) of the inventive method, the ester products may be produced as shown in Reaction 6. The mixture of aliphatic C14 - C24 carboxylic acids produced from suberin and/or cutin by hydrolysis is reacted with at least one saturated or unsaturated, cyclic, aromatic or linear carboxylic anhydride to give a mixture of oligo acids, followed by the esterification thereof, both the hydroxy and the epoxy groups of the carboxylic acids thus yielding esters or semi-esters corresponding to the anhydride used in the reaction.

A simplified model example of the embodiment c) of the invention is shown by Reaction 6 below.

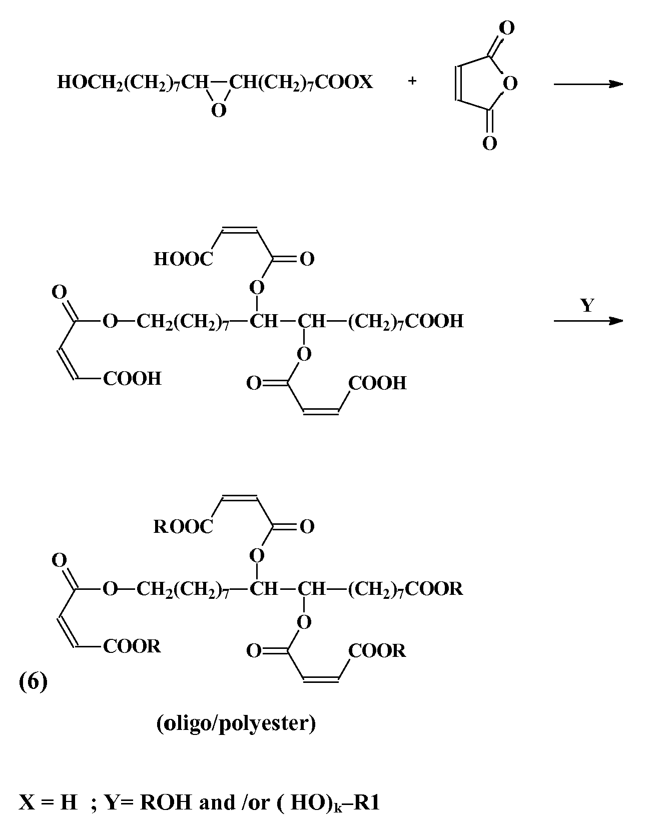

The reaction of the mixture of aliphatic C14 - C24 carboxylic acids obtained from suberin and/or cutin by hydrolysis, with an anhydride is carried out at 60 - 160 °C, in the presence of an acid catalyst or without a catalyst, in an inert solvent such as toluene, xylene, diethylether or a mixture thereof or without any solvent, the reaction time being 1-12 hours, preferably 2-6 hours, thus yielding a mixture of oligoacids as the product. The excessive reactant is decomposed with water and removed from the reaction mixture by washing with a base. The acid catalyst is selected from the group consisting of protonic acid catalyst such as H₂SO₄, MeSO₃H, HCl, H₃PO₄, p-toluenesulphonic acid or Lewis acid catalysts e.g. titanates, (RO)₄Ti or stannous oxide (SnO). The anhydride is used in an amount ranging between 0.7 and 1.3 mol, preferably between 0.9 and 1.1 mol for each free OH-group in the acid mixture of suberin or cutin, assuming that one epoxide group corresponds to two OH groups. Suitable saturated or unsaturated cyclic aromatic or linear carboxylic anhydrides include acetic anhydride and cyclic anhydrides, for instance preferably maleic anhydride, succinic anhydride, and phthalic anhydride and derivatives thereof such as C1-C22 alkyl or alkylensuccinic anhydride, trimellitic anhydride and itaconic anhydride. The intermediate thus obtained is either esterified with a mono- or an oligoalcohol in accordance with the application.

The final product, the oligo/polyester, is produced by reacting said mixture of oligoacids with at least one linear or branched C3-C18 monoalcohol ROH, preferably with 1-, 2- or i-butanol, 1- or i-valeric alcohol, 1-hexanol or 2-ethyl hexanol, and/or with an oligoalcohol (HO)ₖ-R1, where k ≥ 2, said oligoalcohol being selected from the group consisting of C2-C6-diols and polyols such as ethyleneglycol, di- and oligoethyleneglycol, propyleneglycol, di- and oligopropyleneglycol, neopentylglycol, trimethylolpropane, pentaerythritol, dipentaerythritol, 1,4-butanediol and 1,6-hexanediol, either as such or in th epresence of a solvent selected from the group consisting of toluene, xylene and the mixtures thereof, in the presence of a protonic acid catalyst such as H₂SO₄, MeSO₃H, HCl, H₃PO₄, p-toluenesulphonic acid or a Lewis acid catalyst e.g. titanates, (RO)₄Ti or stannous oxide (SnO), at the temperature of 60 - 280 °C, preferably at 100 - 260 °C, in an inert atmosphere, preferably in a nitrogen or argon atmosphere, for 1 - 24 hours, preferably for 2 - 12 h. The mixture is cooled, washed, the solvent and residual monomers are removed for instance by vacuum distillation, followed by drying of the ester product.

Applications of the ester products produced by the method of the invention include industrial products such as lubricants for instance in engines, in oils for refrigerating devices, and in other machines and apparatuses, and further, so-called lubricating additives is fuels, plasticizers, surface active agents, media in cosmetic applications, compatibility improvers for producing mixtures of plastics such as blends or composites, and agents for modifying wood, binders in coatings and printing inks, and in composite structures either as such or in combination with products produced using known techniques.

As composites, particularly biodegradable products may be mentioned, where the products of the invention may be substituted for prior art products based on formaldehyde, since formaldehyde released to the environment after ageing and wetting of said products is hazardous to health. In addition, the products of the invention may be used in aqueous biological composites to replace products based on adipic and sebacic acids.

The oligo- and polyesters produced by the method of the invention may be used as binders and compatibility improvers for producing composites from natural materials such as from cellulose, wood, linen, hemp, starch, and other native fibers, or combinations thereof with known additives, as necessary.

The method of the invention has several advantages. The invention allows for the selective simultaneous (*in situ*) etherification and esterification of the hydroxy and epoxy groups present in mixtures of fatty acids or in mixtures of alkali metal salts thereof obtained from suberin and/or cutin by hydrolysis to give corresponding ethers such as methyl ethers and methyl esters, respectively. The hydroxy and epoxy groups of the carboxylic acids of suberin and cutin being thus etherified, the subsequent reaction (esterification or transesterification) only takes place at the carboxylic acid moiety. Low-cost products may be readily produced in a simple way from mixtures of fatty acids obtained from suberin and/or cutin by hydrolysis, and further, said products may be taylored as desired by modifying with a monoacid and/or alcohol, and so for instance the viscosity of the product may be adjusted e.g. by means of a monoacid. Moreover, controlling the reactions is easy.

It is surprising that the aliphatic carboxylic acids, particularly hydroxy and epoxy acids of suberin and/or cutin may be simultaneously subjected to *in situ* etherification of hydroxy and epoxy groups, and esterification of the carboxylic acids using dimethyl sulphate to give a simple *in situ* intermediates that may be used for the production of ester products in significant volumes by transesterification for instance for coating, painting, fuel and lubricating and additive applications, said ester products also having a significant business potential.

With the products produced according to the method of the invention, natural alternatives to known product blends normally produced from petrochemical starting materials, and to products based on raw oil, potentially containing carcinogens, are achieved.

The invention is now illustrated in more specific manner by the following examples without wishing to limit the scope thereof.

### Examples

### Example 1

### Separation and hydrolysis of suberin

Air-dried bark was cut in strips, granulated and ground to give a powder having particles of 20 mesh, followed by extraction of said powder for 24 hours with aceton in a Soxhlet apparatus. The remaining solid material was filtered and dried. The solid material (100 g) was refluxed in basic 2-propanol (22 g/0.55 mol NaOH in 1 liter of alcohol) for 1 hour. The solid material was filtered from the solution while still hot. The solution was still refluxed for 15 min. The solution was kept in a freezer at least 24 hours. The precipitate was filtered and dried. The product containing sodium salts of carboxylic acids of suberin was a yellowish powder.

### Example 2

### Preparation of suberin acids

The hydrolysis product of suberin (6 g) obtained in Example 1 was dissolved in water (750 ml) in a bath at about 100 °C, followed by cooling the solution. 0.25 M sulphuric acid was added to the solution to adjust the pH of the solution between 2 and 3. The mixture was extracted with diethyl ether (400 + 200 + 200 ml) and dried with sodium sulphate. The solvent was removed by means of a rotary evaporator, followed by drying of the product in vacuum at room temperature. The product contained fatty acids of suberin, the yield thereof being between 84 and 90 %. The product was a yellowish powder.
¹H NMR (ppm): 1.0-1.6(m) CH₂; 2.0 CH₂; 2.2(t) CH₂CO₂; 2.8 CH(O)CH; 3.2 CH(OH)CH(OH); 3.4(t) CH₂OH 3.8 CH(OH); 4.0, 4.2 OH; 5.3 CH=CH; 11.8 OH ¹³C NMR (ppm): 24-28(5s) CH₂; 29(m), 32 CH₂; 34 CH₂COOH; 37 CH₂CH(OH); 56 CH(O)CH; 61 CH₂OH; 70 CH(OH); 73 CH(OH)CH(OH); 130 CH=CH; 174 COOH

Contents of Fatty acid content of suberin is shown in Table 4, by NMR analysis.

**Table 4. Fatty acids of suberin**

| **Monomer** | **% by weight** |
|---|---|
| HOOC(CH₂)₇CH=CH(CH₂)₇COOH + HOCH₂(CH₂)₇CH=CH(CH₂)₇COOH | 16 |
| 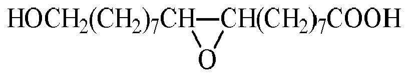 | 37 |
| 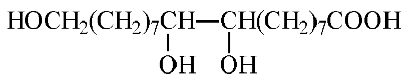 | 18 |
| HOCH₂(CH₂)₂₀COOH + HOOC(CH₂)₂₀COOH | 17 |
| HOCH₂(CH₂)₆CHOH (CH₂)₇COOH | 2 |

### Example 3

### In situ esterification/etherification

The hydrolysis product produced in example 1 (10 g) and K₂CO₃ (9.9 g) were weighed and introduced into a flask. 120 ml of acetone and 6.8 ml of Me₂SO₄ were added. The solution was refluxed for 4 hours, followed by cooling thereof. 11 ml of water was added dropwise, and then the mixture was still agitated for 2 hours. The precipitate was filtered off, and acetone was removed from the solution by means of a rotary evaporator. The product was dissolved in 200 ml of ether, followed by washing with water (3x50 ml). The solution was dried over Na₂SO₄ over night. The desiccant was filtered off, and the solvent was removed by means of a rotary evaporator. Yield of the raw material was about 100 %. The product was a yellow paste, having a boiling range between 175 and 248 °C (GC). NMR analysis showed the presence of the *in situ* esterification/etherification product.
¹H NMR (ppm): 0.9-1.6(m) CH₂; 1.9 CH₂; 2.0 CH₂; 2.7 CH(O)CH; 3.2-3.4 C-OCH₃; 3.5 OCH₃; 3.8 O₂CH₃; 5.2 CH=CH
¹³C NMR (ppm): 24-28(5s) CH₂; 30(m) CH₂; 32-34(4s) CH₂; 51 O₂CH₃; 57 CH(O)CH; 59 OCH₃; 70-74(6s) 82 C-OCH₃; 130 CH=CH

### Example 4

### Transesterification of the in situ product with 2-ethylhexanol

The ether/ester mixture (2 g) prepared in example 3 was weighed and introduced into a flask. 2-ethylhexanol and the catalyst (20 mg of KOH in 5.3 ml of alcohol) were added. The solution was mixed at 70 - 80 °C for 2 hours. The solution was washed with water (3 x 10 ml). Excessive 2-ethylhexanol was distilled off by vacuum distillation. Yield of the product was 98 %. The product was a golden brown oil having a boiling point > 180 °C. NMR analysis showed the succesfull transesterification with 2-ethylhexanol.
¹H NMR (ppm): 0.6-0.9 CH₃; 1.1-1.6(m), 1.9 CH₂; 2.2(t) CH₂CO₂; 3.0 CH(O)CH; 3.2-3.6 C-OCH3; 3.9 CH₂O₂C; 5.3 CH=CH
¹³C NMR (ppm): 11-14(3s) CH₃; 22-27, 32-34 CH₂; 38 C(CH₂)₃; 51 O₂CH₃; 66 CH₂O₂C; 70-75(6s) C-OC; 130 CH=CH; 174, 176, 179 CO₂

### Example 5

### Use of suberin acids as starting materials in the production of polyesters (complex esters)

The acid mixture (5 g) produced in example 2 was weighed and introduced into a flask. Valeric acid (1.7 ml) and toluene (650 ml) were added. The solution was refluxed for 0.5 hours while water was removed. p-Toluenesulphonic acid (50 mg) was added to the flask and refluxing was continued for 5 hours. Bubbling with argon was maintained during the whole refluxing. The solution was allowed to cool, followed by first washing with 10 % Na₂CO₃, and then twice with water. Toluene was removed from the clear yellow phase using a rotary evaporator. The product was dried in vacuum at room temperature. Yield was 5 %, by moles, relative to the starting material. The product was a very viscous yellow oil, the yield thereof being 31 %, by moles. NMR analysis showed that a new ester product was formed.
¹H NMR (ppm): 0.7-1.0 CH₃; 1.1-1.7(m), 2.0 CH₂; 2.3 CH₂CO₂; 2.9 CH(O)CH; 3.6(t) CH₂OH 3.9 CH₂O₂C; 5.3 CH=CH
¹³C NMR (ppm): 13-15 CH₃; 17-30, 32-33 CH₂; 36-42 C(CH₂)₃; 57 CH(O)CH; 62 CH₂OH; 63 CH₂O₂C; 72, 78 C-O; 129 CH=CH; 173, 178 CO₂

### Example 6

### Use of suberin acid mixture as starting material in the production of an alkyd resin

Alkyd resin was produced from tall oil fatty acids (350 g), fatty acids of suberin produced according to example 2 (15 g, 4 % by weight of the total amount of the fatty acids), iso-phthalic acid (55,9 g), and trimethylol propane (89,6 g). All starting materials were weighed into a reaction vessel (glass reactor of 1 liter). The reactor was equipped with a thermal bath, sensors for measuring the temperature, mechanical stirrer, water separation funnel connected to a condenser, and a capillary tube for bubbling an inert gas (N₂) to the reaction mixture. The reaction mixture was mixed and heated at 220 - 260 °C. Progress of the reaction was followed by taking samples every 0.5 - 1 hours. First the acid number and later also the viscosity, as the reaction mixture became clear were determined from the samples (R.E.L cone/plate rotation viscosimeter). The reaction mixture was boiled for 8.5 hours until the acid number and viscosity of the final product were reached. The cooled product was filtered and weighed (411 g). Acid number (15.6) and viscosity, 1175 cP, (Brookfield Synchro Lectic viscosimeter, model LVF, cylinder LV4) of the final product were determined.

### Example 7

### Production of maleic acid semi-esters of suberin fatty acids

Fatty acid mixture of suberin (5.0 g, prepared as shown in example 2), and maleic anhydride (3.0 g/ 2 eq.) were mixed at 80 °C for 3 hours. The reaction mixture was dissolved in diethyl ether (50 ml) and washed with water (50 ml), 2.5 % solution of NaHCO₃ (50 ml) and with water (50 ml). Organic phase was dried over Na₂SO₄, followed by careful evaporation to dryness. Dark yellow viscous liquid was obtained as the product (4.4 g / 60 %). NMR spectrum confirmed the formation of the desired mixture of maleic acid semi-esters of suberin fatty acids.
¹H NMR (300 MHz, DMSO): δ ppm 1.19-1.34 (m, C**H**₂), 1.46-1.51 (m, C**H**₂), 1.56-1.60 (m, C**H**₂), 1.97 (quart, =CHC**H**₂), 2.18 (quart, C**H**₂CO₂H), 3.21 (m, C**H**OH), 4.07 (t, C**H**₂O) 4.71 (m, C**H**O), 4.81 (m, C**H**O), 5.27-5.37 (quint, =C**H**CH₂), 6.30-6.39 (2d, =C**H**CO₂) 6.67-6.85 (m, =C**H**CO₂)
¹³C NMR (300 MHz, DMSO): δ ppm 24.76-28.18 (4, **C**H₂), 28.85-29.33 (m, **C**H₂), 33.91 (**C**H₂CO₂H), 64.71 (**C**H₂O), 70.58 (**C**HO), 73.40 (**C**HOH), 77.64 (**C**HO), 128.94 (=**C**HCO₂), 129.84 (=**C**HCH₂), 131.57 (=**C**HCO₂H) 165.51 (**C**O₂H), 166.71 (**C**O₂CH₂), 174.72 (CH₂**C**O₂H)
APT (300 MHz, DMSO): δ ppm 24.76-28.18 (**C**H₂), 28.85-29.33 (**C**H₂), 33.91 (CH₂), 64.71 (**C**H₂), 70.58 (**C**H), 73.40 (**C**H), 77.64 (**C**H), 128.94 (=**C**H), 129.84 (=**C**H), 131.57 (=**C**H), 165.51 (**C**), 166.71 (**C**), 174.72 (**C**)

### Example 8

### Production of succinic acid semi-esters of suberin fatty acids

Fatty acid mixture of suberin (5.0 g, prepared as shown in example 2), and succinic anhydride (3.1 g/ 2 eq.) were mixed at 130 °C for 2 hours. The reaction mixture was dissolved in diethyl ether (60 ml) and washed with water (30 ml), 2.5 % solution of NaHCO₃ (30 ml) and with water (30 ml). Organic phase was dried over Na₂SO₄, followed by careful evaporation to dryness. Dark yellow viscous liquid was obtained as the product (4.5 g / 62 %). NMR spectrum confirmed the formation of the desired mixture of succinic acid semi-esters of suberin fatty acids.
¹H NMR (300 MHz, DMSO): δ ppm 1.17-1.31 (m, C**H**₂), 1.45-1.55 (m, C**H**₂), 1.97 (quart, =CHC**H**₂), 2.18 (t, C**H**₂CO₂H), 2.44-2.48 (m, CH₂CO₂ + CH₂CO₂H), 3.99 (t, C**H**₂O), 4.69 (m, C**H**O), 4.91 (m, C**H**O), 5.32 (quint, =C**H**), 12.00-12.19 (m, O**H**)
¹³C NMR (300 MHz, DMSO): δ ppm 24.7-28.3 (4s, **C**H₂), 28.9 (m, **C**H₂), 29.3 (m, **C**H₂), 33.9 (**C**H₂CO₂H), 64.1 (**C**H₂O), 70.5 (CHOH), 73.4 (**C**HO), 129.8 (=**C**H), 172.3 (CH₂**C**O₂CH₂), 173.6 (CH₂**C**O₂H), 174.7 (CH₂CH₂**C**O₂H).

### Example 9

### Production of phthalic acid semi-esters of suberin fatty acids

Fatty acid mixture of suberin (5.0 g, prepared as shown in example 2), and phthalic anhydride (3.4 g/ 1.5 eq.) were mixed at 140 °C for 2 hours. Water (50 ml) was added to the reaction mixture and mixing was continued for 20 min. Diethyl ether (50 ml) was added to the reaction mixture, followed by washing thereof with a 2.5 % solution of NaHCO₃ (40 ml) and with water (50 ml). Organic phase was dried over Na₂SO₄, followed by careful evaporation to dryness. Very viscous brown liquid was obtained as the product (5.5 g / 65 %). NMR spectrum confirmed the formation of the desired mixture of phthalic acid semi-esters of suberin fatty acids.
¹H NMR (300 MHz, DMSO): δ ppm 1.21-1.68 (m, C**H**₂), 1.97 (m, =CHC**H**₂), 2.18 (t, C**H**₂CO₂H), 4.20 (t, C**H**₂O), 4.91 (m, C**H**O), 5.32 (m, =C**H**CH₂), 7.63 (m, =C**H**-), 7.75 (m, =C**H**-)
¹³C NMR (300 MHz, DMSO): δ ppm 24.4-27.8 (4, **C**H₂), 29.0 (m, **C**H₂), 33.6 (**C**H₂CO₂H), 65.1 (**C**H₂O), 73.1 (**C**HO), 128.1 (=**C**H-), 128.7 (=**C**H-), 129.5 (=**C**HCH₂), 130.9 (=**C**H-), 131.2 (=**C**H-), 132.1 (=**C**H-), 132.4 (=**C**H-), 167.5 (**C**O₂CH₂), 168.0 (**C**O₂H), 174.4 (CH₂**C**O₂H)

### Example 10

### Production of a mixture of fatty acid acetates of suberin

Fatty acid mixture of suberin (5.0 g, prepared as shown in example 2), and acetic anhydride (2.2 ml / 1.5 eq.) were mixed at 80 °C for 4.5 hours. Water (50 ml) was added to the reaction mixture and mixing was continued for 20 min. Diethyl ether (50 ml) was added to the reaction mixture, followed by washing thereof with a 2.5 % solution of NaHCO₃ (50 ml) and with water (50 ml). Organic phase was dried over Na₂SO₄, followed by careful evaporation to dryness. Yellow viscous liquid was obtained as the product (3.5 g / 53 %). Cone/plate viscosity of the product was 130 cP/50 °C, and 20 cP/100 °C. NMR spectrum confirmed the formation of the desired mixture of fatty acids acetates of suberin.
¹H NMR (300 MHz, CDCl₃): δ ppm 1.25-1.37 (m, C**H**₂), 1.49 (m, C**H**₂), 1.62 (m, C**H**₂), 2.01-2.10 (m, =CHC**H**₂; s, C**H**₃), 2.34 (t, C**H**₂CO₂H), 2.44 (t, C**H**₂CO₂H), 2.91 (m, H**C**OC**H**), 3.40 (m, C**H**OH), 3.58 (m, C**H**₂OH), 4.05 (t, C**H**₂O), 4.83 (m, C**H**O), 5.34 (quint, =C**H**)
¹³C NMR (300 MHz, CDCl₃): δ ppm 21.0 (2, **C**H₃), 24.1-28.5 (10, **C**H₂), 29.1-29-6 (m, **C**H₂), 30.6 (**C**H₂), 34.9 (**C**H₂CO₂H), 35.2 (**C**H₂CO₂H), 57.2 (H**C**O**C**H), 64.6 (**C**H₂O), 72.4-74.5 (2, **C**HOH/CHO), 129.8 (m, =**C**H), 171.3 (**C**O₂), 179.1 (**C**O₂H)

### Example 11

### Esterification of the mixture of maleic acid semi-esters of suberin fatty acids with n-butanol

Mixture of maleic acid semi-esters of suberin fatty acids prepared in example 7 (2.0 g/0.0042 mol) was weighed and introduced into a flask. n-Butanol (6.1 ml/0.0661 mol) and toluene were added into the flask. The mixture was refluxed while removing water for 0.5 hours. p-Toluene sulphonic acid monohydrate (22 mg/l %, by mol) was added into the flask, and refluxing was continued for 5 hours. The solution was allowed to cool, followed by washing twice with 10 % Na₂HCO₃, then twice with water. Phases were separated, and the product phase was dried over Na₂SO₄ over night. Toluene and excess of n-butanol were removed by rotary evaporator. Yield was over 57 %. The product was yellow viscous oil having a mp. of 15 °C, and a bp. of > 180 °C. NMR spectrum confirmed that the desired product was obtained.
¹HNMR(ppm): 0.8-1.0(t) CH₃; 1.2-1.8(m) CH₂; 2,0 CH₂CH=; 2.2-2.3(t) CH₂CO₂; 3.6(t) CH₂OH (alcohol); 4.0(t) CH₂O₂C (suberin); 4.2(t) CH₂CO₂ (maleate); 5.3(t) CH= (suberin); 6.2 CH= (maleate); 6.8 CH= (maleic acid)
¹³CNMR(ppm): 14 CH₃; 19 CH₂CH₃; 24-34(m) CH₂; 35 CH₂CH₂OH (alcohol); 64 CH₂O₂ (suberin); 65 CH₂O₂ (maleate); 65 CH₂OH (alcohol); 71-74(m) C-O; 130 CH= (maleate+suberin); 165 CO₂ (maleate); 174 CO₂ (suberin)

### Example 12

### Esterification of the mixture of maleic acid semi-esters of suberin fatty acids with 2-ethylhexanol

Mixture of maleic acid semi-esters of suberin fatty acids prepared in example 7 (3.0 g) and 2-ethylhexanol (6.0 ml/6.1 eq.) were mixed in argon atmosphere at 150 °C for 15 min. Methylsulphonic acid (0.5 ml / 1.2 eq.) were added to the reaction mixture, and mixing was continued for 5 hours. Water was removed from the mixture by means of toluene, followed by removal of toluene by distillation under reduced pressure. The product was dissolved in diethyl ether (50 ml) and washed with a solution of Na₂HCO₃, and with water (50 ml). Phases were separated, the organic phase was dried over Na₂SO₄ and evaporated to dryness. Brown liquid was obtained as the product (6.0 g) containing minor amounts of 2-ethylhexanol*, and an ester of maleic acid** as a by-product. Based on a DSC analysis, the pour point of the product was below -20 °C, the boiling interval being from 260 to 320 °C. Cone/plate viscosity was 10 cP/50 °C. NMR spectrum confirmed that the desired product was obtained.
¹H NMR (300 MHz, CDCl₃): δ ppm 0.87-0.92 (t, C**H**₃), 1.25-1.41 (m, C**H**₂), 1.53-1.69 (m, C**H**₂), 2.02 (quart, =CHC**H**₂), 2.27-2.40 (t+m, C**H**₂CO₂H), 3.55 (d, CH₂O*), 3.61-3.66 (t, CH₂C**H**₂O₂CCH₂), 3.98 (d, CH₂C**H**₂O₂CCH=), 4.10 (2d+m, CHC**H**₂O₂CCH=), 5.34 (quint, =C**H**CH₂), 6.23 (s, =C**H**CO₂), 6.75 (s, =C**H**CO₂**)
¹³C NMR (300 MHz, CDCl₃): δ ppm 10.8-11.1 (3, **C**H₃), 14.0 (2, **C**H₃), 22.9-23.8 (5, **C**H₂), 28.9-30.4 (7, **C**H₂), 34.4 (**C**H₂CO₂H), 38.6-38.7 (2, **C**H), 41.9 (**C**H*), 65.2 (**C**H₂OH*), 66.6 (CH₂**C**H₂O₂CCH=), 67.7 (CH**C**H₂O₂CCH= + CH₂**C**H₂O₂CCH₂), 72.1 (**C**HO/**C**HOH), 77.2 (**C**HO), 129.7 (2, =**C**HCH₂ + =**C**HCO₂), 133.6 (=**C**HCO₂**), 165.4 (=CH**C**O₂), 174.1 (m, **C**O₂CH₂, **C**O₂H)

## Claims

1. Method for producing oligo- and polyesters of mixtures of aliphatic C14 - C24 carboxylic acids, **characterized in that** mixtures of aliphatic C14 - C24 carboxylic acids obtained from suberin and/or cutin by hydrolysis are esterified according to embodiment c), the embodiment
c) comprising the reaction of said mixtures of aliphatic C14 - C24 carboxylic acids obtained from suberin and/or cutin by hydrolysis with at least one saturated or unsaturated, cyclic, aromatic or linear carboxylic anhydride to give a mixture of oligoacids, followed by the reaction thereof with at least one linear or branched C3-C18 monoalcohol and/or a oligoalcohol, in the presence of an acid catalyst.

2. Method according to claim 1, **characterized in that** in the embodiment c), said saturated or unsaturated cyclic aromatic or linear carboxylic anhydride is selected from the group consisting of acetic anhydride, maleic anhydride, succinic anhydride, phthalic anhydride, C1-C22 alkyl or alkylensuccinic anhydride, trimellitic anhydride, and itaconic anhydride.

3. Method according to claim 1 or 2, **characterized in that** in the embodiment c), the reaction with said carboxylic anhydride is carried out at a temperature of 60 - 160 °C, in the presence of an acid catalyst or without a catalyst, in an inert solvent, preferably toluene or xylene, or a mixture thereof, or without a solvent.

4. Method according to any one of claims 1 - 3, **characterized in that** in the embodiment c), the mixture of oligoacids is reacted with a monoalcohol selected from the group consisting of 1-, 2- and i-butanol, 1- and i-valeric alcohol, 1-hexanol and 2-ethylhexanol, and/or with an oligoalcohol selected from the group consisting of C2-C6-diols, and polyols, preferably with ethyleneglycol, di- and oligoethyleneglycol, propyleneglycol, di- and oligopropyleneglycol, neopentylglycol, trimethylolpropane, pentaerythritol, dipentaerythritol, 1,4-butanediol and 1,6-hexanediol.

5. Method according to any one of claims 1 - 4, **characterized in that** in the embodiment c), the mixture of oligoacids is reacted either as such or in a solvent selected from the group consisting of toluene, xylene and mixtures thereof, in the presence of a protonic acid catalyst selected from the group consisting of H₂SO₄, MeSO₃H, HCl, H₃PO₄, p-toluene sulphonic acid or Lewis acid catalysts, at a temperature of 60 - 280 °C, preferably at 100 - 260 °C, in an inert atmosphere, preferably in a nitrogen or argon atmosphere.

6. Use of the oligo- and polyesters of mixtures of aliphatic C14 - C24 carboxylic acids, and mixtures thereof, produced with the method according to claim 1 for the production of alkyd resins.

7. Use of the oligo- and polyesters of mixtures of aliphatic C14 - C24 carboxylic acids, and mixtures thereof, produced by the method according to claim 1 in lubricant, fuel component, plasticizer, binder, compatibility improver, coating, composite, adhesive, printing ink, and cosmetic applications and as an agent for modifying wood, and as surface active agents.
